# FASCICULE DE BREVET EUROPEEN

(11) **EP 1 677 779 B1**
(45) Date de publication et mention de la délivrance du brevet: **24.09.2008**
(21) Numéro de dépôt: 04791453.6
(22) Date de dépôt: 01.10.2004
(51) Int. Cl.: A61K 31/18, A61K 31/4365, A61P 9/00

(54) **ASSOCIATION D'UN ANTAGONISTE DES RECEPTEURS TP ET DE CLOPIDOGREL**
KOMBINATION EINES ANTAGONISTEN DER TP-REZEPTOREN MIT CLOPIDOGREL
COMBINATION OF AN ANTAGONIST OF THE TP-RECEPTORS AND CLOPIDOGREL

(30) Priorité: 03.10.2003 FR 0311595
(43) Date de publication de la demande: 12.07.2006
(73) Titulaire: Les Laboratoires Servier, 92415 Courbevoie Cédex (FR)
(72) Inventeur: CLOAREC-BLANCHARD, Laure, F-75015 Paris (FR); CORDA, Stefano, F-75011 Paris (FR); LEROND, Laurence, F-78160 Marly-Le-Roi (FR)
(86) Numéro de dépôt international: PCT/FR2004/002489
(87) Numéro de publication internationale: WO 2005/032533

(56) Documents cités:
- EP-A- 0 648 741
- US-A1- 2003 109 543
- KAKKOS S.K. ET AL: "S- 18886: Servier." CURRENT OPINION IN INVESTIGATIONAL DRUGS, (1 SEP 2002) 3/9 (1324-1327)., 1 septembre 2002 (2002-09-01), XP008028385
- ANDERSON H V ET AL: "Platelet inhibition reduces cyclic flow variations and neointimal proliferation in normal and hypercholesterolemic-atherosclerotic canine coronary arteries." CIRCULATION. 6 NOV 2001, vol. 104, no. 19, 6 novembre 2001 (2001-11-06), pages 2331-2337, XP002313743 ISSN: 1524-4539
- YAO S K ET AL: "Combined ADP and thromboxane A2 antagonism prevents cyclic flow variations in stenosed and endothelium-injured arteries in nonhuman primates." CIRCULATION. UNITED STATES DEC 1993, vol. 88, no. 6, décembre 1993 (1993-12), pages 2888-2893, XP009028066 ISSN: 0009-7322
- HERMAN A G: "Rationale for the combination of anti-aggregating drugs" THROMBOSIS RESEARCH 15 SEP 1998 UNITED KINGDOM, vol. 92, no. 1 SUPPL. 1, 15 septembre 1998 (1998-09-15), pages S17-S21, XP000910424 ISSN: 0049-3848

## Description

La nouvelle invention a pour objet l'association d'un antagoniste spécifique des récepteurs TP et de clopidogrel.

Le thromboxane A₂ (TXA₂) est un métabolite instable de l'acide arachidonique qui est impliqué dans la pathogenèse de nombreuses maladies cardiovasculaires. Le thromboxane A₂ est un puissant activateur des plaquettes mais également un puissant vasoconstricteur qui possède des propriétés prolifératives et pro-adhésives cellulaires.

Le TXA₂ et d'autres métabolites de l'acide arachidonique tels que les endoperoxydes (PGG₂-PGH₂), les HETE et les isoprostanes exercent leur activité par le biais de récepteurs communs nommés récepteurs TP (thromboxane - prostaglandines - endoperoxydes).

La demande de brevet US 2003/0109543 décrit l'effet antiplaquettaire synergique d'une association de clopidogrel et d'ifetroban, un antagoniste du TXA₂, dans un modèle de thrombose reineuse.

Récemment, de nombreux travaux de recherche ont été effectués dans le but de prévenir les phénomènes liés à la production excessive de thromboxane A₂ dans les systèmes cardiovasculaire et neurovasculaire. Parmi ces antagonistes, ceux décrits dans le brevet EP 648 741 se sont avérés de puissants et sélectifs antagonistes des récepteurs TP, actifs par voie orale et ayant une longue durée d'action.

Plus particulièrement, le composé (A) de formule (I): 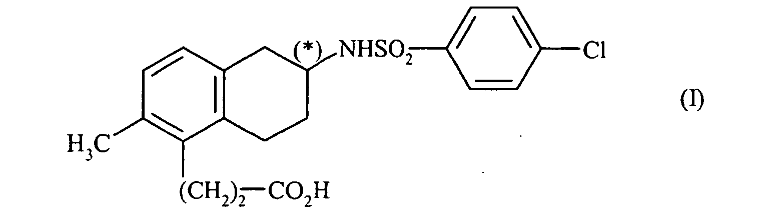 sous forme racémique ou d'isomère optiquement pur ainsi que ses sels d'addition pharmaceutiquement acceptables, s'est avéré être un puissant anti-athérothrombotique.

Le composé A est un antagoniste spécifique des récepteurs TP, plus particulièrement un antagoniste spécifique du thromboxane A₂ et des récepteurs des prostaglandines-endoperoxides (PGG₂-PGH₂) qui lui confère un puissant effet athérothrombotique.

De manière générale, la formation d'un thrombus après la rupture d'une plaque d'athérome résulte de l'interaction entre les plaquettes circulantes et le collagène de la lame basale de l'endothélium vasculaire exposée au flux sanguin. Ce phénomène est nommé athérothrombose.

Le collagène est présent dans la lame basale de la paroi vasculaire et est le facteur déterminant de la thrombogénicité des lésions athéromateuses chez l'homme comme chez l'animal.

L'adhésion plaquettaire aux fibres du collagène intervient via le récepteur au collagène et implique l'adhésion des plaquettes, leur activation et leur agrégation.

L'activation des plaquettes est accompagnée de la libération de deux principaux agonistes, l'ADP et le thromboxane A₂ qui se lient à leurs récepteurs respectifs (P2Y, TP) sur les plaquettes adjacentes et amplifient l'adhésion et l'agrégation plaquettaire.

L'ADP est également présent dans le sang en tant que médiateur circulant, alors que le thromboxane A₂ est un puissant médiateur secondaire formé dans les plaquettes activées à partir de l'acide arachidonique via la cyclo-oxygenase 1.

Le thromboxane A₂ non seulement favorise la thrombose mais induit également un dysfonctionnement de la paroi vasculaire (vasoconstriction) et favorise la prolifération et l'infiltration inflammatoire de la paroi.

Parmi les traitements antiplaquettaires disponibles actuellement, l'aspirine permet l'inhibition de la production plaquettaire issue du thromboxane A₂, le clopidogrel, quant à lui, inhibe l'agrégation plaquettaire induite par l'ADP.

L'ADP et le thromboxane A₂ jouent un rôle important et complémentaire dans la formation du thrombus artériel.

Le composé A agit en bloquant l'agrégation plaquettaire induite par le thromboxane A₂ et les autres ligands des récepteurs TP, quelle que soit leur origine, plaquettaire ou extraplaquettaire.
Il agit de plus en inhibant la vasoconstriction induite par le thromboxane A₂ et en s'opposant au dysfonctionnement endothélial et à la prolifération ainsi qu'à l'inflammation de la paroi vasculaire.

Nous avons maintenant découvert chez l'Homme que l'association du composé A avec le clopidogrel permettait de façon surprenante d'obtenir une synergie d'activité antithrombotique.
En effet, le composé A et le clopidogrel agissant sur des voies complètement différentes de l'agrégation plaquettaire, il était particulièrement judicieux d'associer ces deux composés afin d'envisager une nouvelle approche thérapeutique.
De manière surprenante, il s'est avéré que l'association du composé A et du clopidogrel permettait d'obtenir une importante synergie d'activité qu'aucun enseignement de la littérature ne pouvait laisser prévoir. Cette association a permis d'améliorer l'effet antithrombotique évalué par l'inhibition de l'agrégation plaquettaire *ex-vivo* induite par le collagène.

Lors de ce test, il a été montré que l'activité antithrombotique du composé A est potentialisée en présence de clopidogrel et augmente de manière extrêmement importante et totalement non prévisible. En outre cette association présente un bon profil d'acceptabilité.

Dans les associations selon l'invention, le composé (A) et le clopidogrel peuvent se présenter sous forme de sels pharmaceutiquement acceptables.

Parmi les sels d'addition du composé (A), on peut citer à titre non limitatif les sels d'addition à une base pharmaceutiquement acceptable comme les sels de sodium, de potassium, de *tert*butylamine, de diéthylamine.

A titre préférentiel, le sel utilisé sera le sel de sodium.

Parmi les sels d'addition du clopidogrel, on préférera l'hydrogénosulfate.

Dans les associations selon l'invention, le composé (A) possède préférentiellement la configuration absolue (R).

La présente invention concerne également les compositions pharmaceutiques renfermant une association du composé (A) et de clopidogrel, éventuellement sous forme de sels pharmaceutiquement acceptables avec un ou plusieurs excipients inertes, non toxiques et appropriés.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les suppositoires, les crèmes, pommades, gels dermiques.

La posologie est adaptable selon la nature et la sévérité de l'affection, la voie d'administration ainsi que l'âge et le poids du patient.

Dans les compositions selon l'invention, les quantités des principes actifs sont comprises entre 1 et 300 mg pour le composé (A) et entre 10 et 600 mg pour le clopidogrel.

Les compositions selon l'invention sont donc utiles pour le traitement des maladies cardiovasculaires dans lesquelles l'activation des récepteurs TP est impliquée ainsi que pour le traitement des conséquences de ces maladies. Ces conditions incluent le syndrome coronaire aigu, l'angor stable ou instable, le dysfonctionnement endothélial, les maladies vasculaires liées à l'athérosclérose, l'hypertension, le diabète, l'insuffisance cardiaque, la prévention et le traitement des troubles du système vasculaire, cardiovasculaire ou neurovasculaire, et des troubles thromboemboliques associés notamment à l'athérosclérose, la fibrillation auriculaire, les gestes chirurgicaux invasifs en cardiologie, neurologie, pathologie vasculaire et radiologie (angioplastie, pose de stents, pontages, cathéters...).

### Mesure de l'inhibition de l'agrégation plaquettaire induite par le collagène :

10 mg du composé A et 75 mg de clopidogrel ont été administrés par voie orale pendant trois jours à 18 volontaires, traités au préalable par 75 mg de clopidogrel pendant 7 jours. L'effet de l'association du composé A et du clopidogrel a été comparé aux effets du composé A et du clopidogrel administrés séparément.
Lors de ce test, le pourcentage d'inhibition de l'agrégation plaquettaire ex-vivo induite par le collagène (5µg/ml) a été calculé par mesure de l'agrégation plaquettaire sur plasma citraté riche en plaquettes (PRPc) à l'aide d'un agrégomètre.

Les résultats obtenus sont les suivants :
- l'administration du composé A seul conduit à 35 % d'inhibition,
- l'administration du clopidogrel seul conduit à 11 % d'inhibition,
- l'administration de l'association du composé A et du clopidogrel conduit à 62 % d'inhibition.

Les résultats montrent très clairement que l'administration de ces deux composés en association permet d'obtenir un effet synergique sur l'agrégation plaquettaire induite par le collagène.
Cet effet antiagrégant obtenu grâce à l'association est ainsi supérieur à la somme des effets des deux produits pris séparément. Rien dans la littérature ne pouvait laisser suggérer ce type de résultat.
Les résultats suggèrent que cette association peut s'avérer bénéfique dans les conditions aiguës ou chroniques où un effet antithrombotique majoré associé à un effet vasculaire est nécessaire (prise en charge en aigu ou prévention secondaire des maladies neurovasculaires ou cardiovasculaires...).

## Revendications

1. Association du composé (A) de formule (I) éventuellement sous forme d'isomère optique ou d'un de ses sels pharmaceutiquement acceptable et de clopidogrel ou d'un de ses sels pharmaceutiquement acceptable :

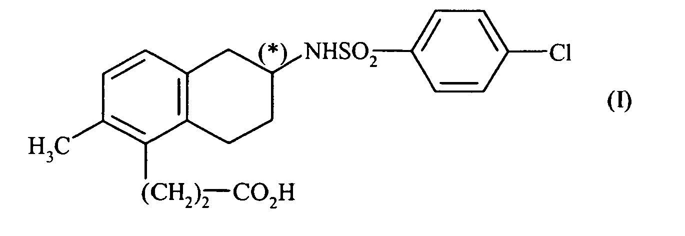

2. Association selon la revendication 1 **caractérisée en ce que** le composé (A) est sous la forme d'isomère optique de configuration (R).

3. Association selon l'une quelconque des revendications 1 ou 2 **caractérisée en ce que** le composé (A) est sous la forme d'un sel de sodium.

4. Association selon l'une quelconque des revendications 1, 2 ou 3 **caractérisée en ce que** le clopidogrel est sous forme d'hydrogénosulfate.

5. Composition pharmaceutique contenant comme principes actifs une association du composé (A) éventuellement sous forme d'isomère optique ou d'un de ses sels pharmaceutiquement acceptables et de clopidogrel ou d'un de ses sels pharmaceutiquement acceptables en combinaison avec un ou plusieurs excipients ou véhicules inertes pharmaceutiquement acceptables.

6. Composition pharmaceutique selon la revendication 5 **caractérisée en ce que** le composé (A) est sous la forme d'isomère optique de configuration (R).

7. Composition pharmaceutique selon l'une quelconque des revendications 5 ou 6 **caractérisée en ce que** le composé (A) est sous forme de sel de sodium.

8. Composition pharmaceutique selon l'une quelconque des revendications 5, 6 ou 7 **caractérisée en ce que** le clopidogrel est sous forme d'hydrogénosulfate.

9. Composition pharmaceutique selon l'une quelconque des revendications 5 à 8 **caractérisé en ce que** les quantités de principes actifs sont respectivement comprises entre 1 et 300 mg pour le composé (A) et 10 et 600 mg pour clopidogrel.

10. Composition pharmaceutique selon l'une quelconque des revendications 5 à 9 le traitement des maladies cardiovasculaires dans lesquelles l'activation des récepteurs TP est impliquée ainsi que pour le traitement des conséquences de ces maladies, sélectionnées parmi le syndrome coronaire aigü, l'angor stable ou instable, le dysfonctionnement endothélial, les maladies vasculaires liées à l'athérosclérose, à l'hypertension, au diabète et à l'insuffisance cardiaque, la prévention et le traitement des troubles du système vasculaire, cardiovasculaire ou neurovasculaire, et des troubles thromboemboliques associés notamment à l'athérosclérose, la fibrillation auriculaire, les gestes chirurgicaux invasifs en cardiologie, neurologie, pathologie vasculaire et radiologie.

## Claims

1. Association of compound (A) of formula (I), optionally in the form of an optical isomer, or one of its pharmaceutically acceptable salts, and clopidogrel or one of its pharmaceutically acceptable salts :

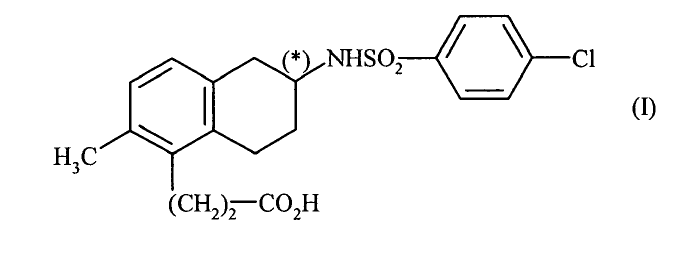

2. Association according to claim 1, **characterised in that** compound (A) is in the form of the optical isomer of (R) configuration.

3. Association according to either claim 1 or claim 2, **characterised in that** compound (A) is in the form of the sodium salt.

4. Association according to any one of claims 1, 2 or 3, **characterised in that** clopidogrel is in the form of the hydrogen sulphate.

5. Pharmaceutical composition comprising as active ingredients an association of compound (A), optionally in the form of an optical isomer, or one of its pharmaceutically acceptable salts, and clopidogrel or one of its pharmaceutically acceptable salts, in combination with one or more pharmaceutically acceptable, inert excipients or carriers.

6. Pharmaceutical composition according to claim 5, **characterised in that** compound (A) is in the form of the optical isomer of (R) configuration.

7. Pharmaceutical composition according to either claim 5 or claim 6, **characterised in that** compound (A) is in the form of the sodium salt.

8. Pharmaceutical composition according to any one of claims 5, 6 or 7, **characterised in that** clopidogrel is in the form of the hydrogen sulphate.

9. Pharmaceutical composition according to any one of claims 5 to 8, **characterised in that** the amounts of active ingredients are in the respective ranges of from 1 to 300 mg for compound (A) and from 10 to 600 mg for clopidogrel.

10. Pharmaceutical composition according to any one of claims 5 to 9 for the treatment of cardiovascular illnesses involving the activation of TP receptors and also for the treatment of the consequences of those illnesses, selected from acute coronary syndrome, stable or unstable angina, endothelial dysfunction, vascular illnesses associated with atherosclerosis, hypertension, diabetes and heart failure, the prevention and treatment of disorders of the vascular, cardiovascular or neurovascular system and of thrombo-embolic disorders associated especially with atherosclerosis, auricular fibrillation and invasive surgical procedures in cardiology, neurology, vascular pathology and radiology.

## Patentansprüche

1. Kombination aus der Verbindung (A) der Formel (I), gegebenenfalls in Form des optischen Isomeren oder eines seiner pharmazeutisch annehmbaren Salze, und Clopidogrel oder eines seiner pharmazeutisch annehmbaren Salze:

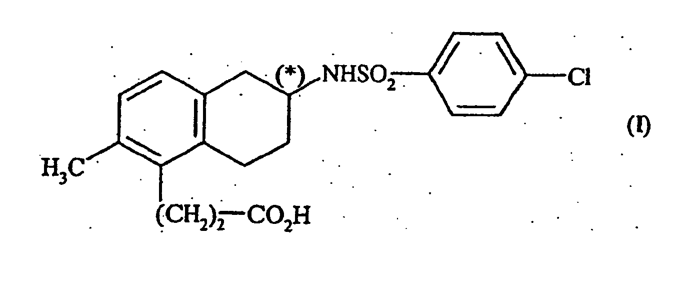

2. Kombination nach Anspruch 1, **dadurch gekennzeichnet, dass** die Verbindung (A) in Form des optischen Isomeren in der Konfiguration (R) vorliegt.

3. Kombination nach einem der Ansprüche 1 oder 2, **dadurch gekennzeichnet, dass** die Verbindung (A) in Form eines Natriumsalzes vorliegt.

4. Kombination nach einem der Ansprüche 1, 2 oder 3, **dadurch gekennzeichnet, dass** Clopidogrel in Form des Hydrogensulfats vorliegt.

5. Pharmazeutische Zubereitung enthaltend als Wirkstoffe eine Kombination aus der Verbindung (A), gegebenenfalls in Form des optischen Isomeren oder eines seiner pharmazeutisch annehmbaren Salze, und Clopidogrel oder eines seiner pharmazeutisch annehmbaren Salze in Kombination mit einem oder mehreren inerten, pharmazeutisch annehmbaren Hilfsstoffen oder Bindemitteln.

6. Pharmazeutische Zubereitung nach Anspruch 5, **dadurch gekennzeichnet, dass** die Verbindung (A) in Form des optischen Isomeren der Konfiguration (R) vorliegt.

7. Pharmazeutische Zubereitung nach einem der Ansprüche 5 oder 6, **dadurch gekennzeichnet, dass** die Verbindung (A) in Form des Natriumsalzes vorliegt.

8. Pharmazeutische Zubereitung nach einem der Ansprüche 5, 6 oder 7, **dadurch gekennzeichnet, dass** Clopidogrel in Form des Hydrogensulfats vorliegt.

9. Pharmazeutische Zubereitung nach einem der Ansprüche 5 bis 8, **dadurch gekennzeichnet, dass** die Mengen der Wirkstoffe zwischen 1 und 300 mg für die Verbindung (A) beziehungsweise 1 und 600 mg für Clopidogrel betragen.

10. Pharmazeutische Zubereitung nach einem der Ansprüche 5 bis 9 zur Behandlung von kardiovaskulären Erkrankungen, bei denen die Aktivierung der TP-Rezeptoren beteiligt ist sowie zur Behandlung von Folgen dieser Erkrankungen ausgewählt aus akutem Herzsyndrom, stabilem oder instabilem Angor, endothelialer Dysfunktion, mit der Atherosklerose, der Hypertension, dem Diabetes und der Herzinsuffizienz verknüpften Gefäßerkrankungen, zur Vorbeugung und Behandlung von Störungen des vaskulären, kardiovaskulären oder neurovaskulären Systems und von thromboembolischen Störungen, die insbesondere mit Atherosklerose, Herzflimmern, invasiven kardiologischen und chirurgischen Eingriffen, der Neurologie, Gefäßpathologie und Radiologie verknüpft sind.
